Europäisches Patentamt

European Patent Office

Office européen des brevets

(19)

(11) **EP 0 617 597 B1**

(12) **EUROPÄISCHE PATENTSCHRIFT**

(45) Veröffentlichungstag und Bekanntmachung des Hinweises auf die Patenterteilung:
**12.03.1997  Patentblatt 1997/11**

(21) Anmeldenummer: 92924688.2

(22) Anmeldetag: **08.12.1992**

(51) Int. Cl.$^6$: **A61F 2/32**

(86) Internationale Anmeldenummer:
**PCT/EP92/02833**

(87) Internationale Veröffentlichungsnummer:
**WO 93/11722 (24.06.1993  Gazette 1993/15)**

(54) **KÜNSTLICHES GELENK**

ARTIFICIAL JOINT

ARTICULATION ARTIFICIELLE

(84) Benannte Vertragsstaaten:
**AT BE CH DE DK ES FR GB GR IE IT LI LU MC NL PT SE**

(30) Priorität: **11.12.1991 DE 4140838**

(43) Veröffentlichungstag der Anmeldung:
**05.10.1994  Patentblatt 1994/40**

(73) Patentinhaber:
• **Theusner, Joachim, Dr.**
  **80539 München (DE)**
• **Kubein-Meesenburg, Dietmar, Prof. Dr.**
  **37547 Kreiensen (DE)**
• **NÄGERL, Hans Dr.**
  **37130 Gleichen (DE)**

(72) Erfinder:
• **KUBEIN-MEESENBURG, Dietmar**
  **D-3350 Kreiensen 1 (DE)**
• **NÄGERL, Hans**
  **D-3407 Gleichen/OT Klein-Lengden (DE)**

(74) Vertreter: **Zapf, Christoph, Dipl.-Ing.**
  **Patentanwälte Dr. Solf und Zapf**
  **Postfach 13 01 13**
  **42028 Wuppertal (DE)**

(56) Entgegenhaltungen:
**WO-A-90/11062          US-A- 3 916 451
US-A- 3 955 568**

EP 0 617 597 B1

**Beschreibung**

Die vorliegende Erfindung betrifft ein künstliches Gelenk zum Ersatz insbesondere von menschlichen Gelenken, bestehend aus mindestens zwei Gelenkteilen mit zueinander sich bewegenden sphärischen Funktionsflächen, wobei die Krümmungsverhältnisse der eine kreisförmige Schnittkontur aufweisenden Funktionsflächen zueinander konvex-konvex, konvex-konkav oder konkav-konkav sind und die Gelenkgeometrie durch eine Gelenkkette mit zwei Gelenkachsen bestimmt ist, die durch die Rotationszentren $M_1$ und $M_2$ der Funktionsflächen mit den Radien $R_1$ und $R_2$ verlaufen, wobei $R_1$ der Radius der kreisförmigen Schnittkontur der Funktonsfläche mit dem Mittelpunkt $M_1$ und $R_2$ der Radius der kreisförmigen Schnittkontur der Funktionsfläche mit dem Mittelpunkt $M_2$ ist, und wobei zwischen den beiden Funktionsflächen der Gelenkteile ein Druckverteilungskörper angeordnet ist, dessen an den Funktionsflächen anliegende Gleitflächen eine den Funktionsflächen entsprechend angepaßte Krümmung aufweisen.

Aus der WO-A-9011062 entsprechend der deutschen Patentanmeldung P 39 08 958.4 ist ein derartiges Gelenk bekannt. Hierbei ist zwischen den beiden Gelenkteilen ein Druckverteilungskörper angeordnet, dessen an den Funktionsflächen anliegende Gleitflächen eine den Funktionsflächen entsprechend angepaßte Krümmung aufweisen. Es hat sich herausgestellt, daß es für die Funktionstauglichkeit dieses Gelenks erforderlich ist, daß der Druckverteilungskörper frei beweglich bleibt.

Der Erfindung liegt die Aufgabe zugrunde, das vorstehende bekannte Gelenk derart zu verbessern, daß in allen Belastungsstellungen des Gelenkes die freie Beweglichkeit des Druckverteilungskörpers gegeben ist. Erfindungsgemäß wird dies dadurch erreicht, daß die Gleitflächen des Druckverteilungskörpers gleich groß gestaltet sind, oder daß eine abweichung zwischen den Flächengrößen der beiden Gleitflächen des Druckverteilungskörpers von maximal 5 - 10% besteht. Somit sind die Kontaktflächen zu beiden Seiten des Druckverteilungskörpers mit den beiden Gelenkteilen im wesentlichen gleich groß Hierdurch treten auf beiden Seiten des Druckverteilungskörpers gleiche Druckbeanspruchungen auf.

Weiterhin ist es vorteilhaft, wenn zudem die Gleitflächen möglichst reibungsfrei sind. Dies wird erfindungsgemäß dadurch erreicht, daß die Gleitflächen insbesondere hochglanzpoliert ausführt sind.

Weiterhin ist es vorteilhaft, wenn die Radien der eine kreisförmige Schnittkontur aufweisenden Funktionsflächen gleich groß sind bzw. nur einen geringen Unterschied aufweisen. Hierdurch ist es möglich, die Gleichheit der Gleitflächen des Druckverteilungskörpers dadurch zu erreichen, daß lediglich eine spezielle Gestaltung seines Randwulstes ausreicht, um die Flächengleichheit herbeizuführen. Weiterhin kann es vorteilhaft sein, wenn die minimale Dicke des Druckverteilungskörpers bei einer Ausbildung der Funktionsflächen der beiden Gelenkteile in der Ausbildung konvex-konkav, die auf der Verbindungslinie der beiden Rotationszentren gegeben ist, klein gegenüber den beiden Radien der Funktionsflächen ist, und zwar vorzugsweise, daß die minimale Dicke des Druckverteilungskörpers kleiner/gleich 50% des kleineren Radius der beiden Funktionsflächen ist. Zudem kann es vorteilhaft sein, wenn der Radius R der überschlagenen, dimeren Kette ca. 2,5 - 6 mm beträgt. Diese Dimensionierung des Radius R ist deshalb zweckmäßig, da hierdurch sicher gewährleistet wird, daß die Friktion zwischen den aufeinander gleitenden Körpern überwunden wird.

Anhand des in den beiliegenden Zeichnungen dargestellten Ausführungsbeispiels wird die Erfindung näher erläutert.

Wie sich aus der beiliegenden Fig. ergibt, besteht ein erfindungsgemäßes Gelenk aus einem Gelenkteil 21 und einem Gelenkteil 22. Die beiden Gelenkkörper 21, 22 besitzen unterschiedliche sphärische Krümmungen. Der Gelenkkörper 21 weist eine konkave Funktionsfläche 23 auf und der Gelenkkörper 22 eine konvexe Funktionsfläche 24.

Zwischen den Gelenkteilen 21, 22 ist ein Druckverteilungskörper 25 angeordnet. Dieser Druckverteilungskörper 25 besitzt Gleitflächen 26, 27. Das Gelenkteil 22 besitzt das Rotationszentrum $M_1$, und die kreisförmige Schnittkontur der Funktionsfläche 24 des Gelenkteils 22 besitzt den Radius $R_1$. Das Gelenkteil 21 besitzt das Rotationszentrum $M_2$, und seine konkave Funktionsfläche 23 weist in ihrer kreisförmigen Schnittkontur den Radius $R_2$ auf. Hierbei sind die Rotationszentren innerhalb des Gelenkteils mit der konvexen Funktionsfläche 24 angeordnet. Der Radius R der Gelenkachsenbahn ergibt sich aus der Beziehung

$$R = R_2 - R_1 - D.$$

Hierbei ist D die Dicke des Druckverteilungskörpers 25 auf der Verbindungslinie der Rotationszentren $M_1$ und $M_2$. Der Radius $R_2$ ist derart bemessen, daß R eine positive Größe ist, so daß $M_2$ in Richtung auf das Gelenkteil 22 gegenüber $M_1$ versetzt ist. Hierbei ergibt sich eine stabile Lage unter Druckbeanspruchung der gleitenden Körper zueinander.

Die Gleitflächen 26, 27 sind gleich groß ausgebildet, so daß die Haftreibungskräfte an beiden Gleitflächen auch gleich groß sind. Dies wird zweckmäßigerweise dadurch erreicht, daß die beiden Radien $R_1$ und $R_2$ der Funktionflächen 23, 24 nicht zu stark differieren, so daß die Flächengleichheit durch die Gestaltung der Randwülste erzielt werden kann. Jedoch kann eine Abweichung der Flächengröße der beiden Gleitflächen von maximal 5 - 10% zugelassen werden, ohne daß hierdurch der gewünschte Effekt wesentlich beeinträchtigt wird.

Die vorstehende Ausgestaltung bewirkt, daß die minimale Dicke D des Druckverteilungskörpers klein gegenüber den Radien der Funktionsflächen ist und zwar soll D maximal etwa 50% des kleineren der beiden Radien $R_1$, $R_2$ sein.

Weiterhin sind die Gleitflächen 25, 26 derart in ihrer Oberfläche beschaffen, daß diese praktisch reibungsfrei sind. Dies kann dadurch erreicht werden, daß die Gleitfläche hochglanzpoliert sind.

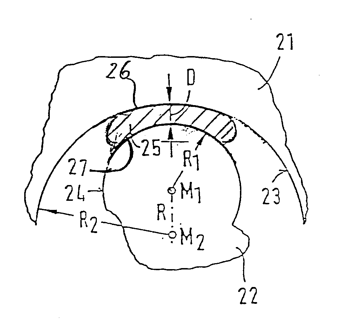

**Patentansprüche**

1. Künstliches Gelenk zum Ersatz insbesondere von menschlichen Gelenken, bestehend aus mindestens zwei Gelenkteilen (21, 22) mit zueinander sich bewegenden sphärischen Funktionsflächen, wobei die Krümmungsverhältnisse der eine kreisförmige Schnittkontur aufweisenden Funktionsflächen (23, 24) zueinander konvex-konvex, konvex-konkav oder konkav-konkav sind und die Gelenkgeometrie durch eine Gelenkkette mit zwei Gelenkachsen bestimmt ist, die durch die Rotationszentren $M_1$ und $M_2$ der Funktionsflächen mit den Radien $R_1$ und $R_2$ verlaufen, wobei $R_1$ der Radius der kreisförmigen Schnittkontur der Funktionsfläche mit dem Mittelpunkt $M_1$ und $R_2$ der Radius der kreisförmigen Schnittkontur der Funktionsfläche mit dem Mittelpunkt $M_2$ ist, und wobei zwischen den beiden Funktionsflächen (23, 24) der Gelenkteile (21, 22) ein Druckverteilungskörper (25) angeordnet ist, dessen an den Funktionsflächen anliegende Gleitflächen (26, 27) eine den Funktionsflächen entsprechend angepaßte Krümmung aufweisen, **dadurch gekennzeichnet**, daß die Gleitflächen (26, 27) des Druckverteilungskörpers (25) gleich groß sind, oder daß eine Abweichung zwischen den Flächengrößen der beiden Gleitflächen (26, 27) des Druckverteilungskörpers (25) von maximal 5-10 % besteht.

2. Gelenk nach Anspruch 1, **dadurch gekennzeichnet,** daß die Gleitflächen (26, 27) hochglanzpoliert sind.

3. Gelenk nach Anspruch 2, **dadurch gekennzeichnet,** daß die Funktionsflächen (23, 24) der Gelenkteile (21, 22) konvex ausgebildet sind und ihre Rotationszentren $M_1$ und $M_2$ in dem zugehörigen Gelenkteil (21, 22) liegen und ihre Gelenkachsenbahn einen Radius $R = R_1 + R_2 + D$ besitzt.

4. Gelenk nach Anspruch 2, **dadurch gekennzeichnet,** daß jeweils eine Funktionsfläche (24) eines Gelenkteiles (22) konvex und die andere Funktionsfläche (23) des Gelenkteils (21) konkav ausgebildet ist und ihre Rotationszentren $M_1$ und $M_2$ innerhalb des Gelenkteiles mit der konvexen Funktionsfläche liegen und ihre Gelenkachsenbahn einen Radius $R = R_2 - R_1 - D$ besitzt, wobei $R_2$ größer ist als die Summe aus $R_1$

und D, so daß eine stabile überschlagene, dimere Kette gegeben ist.

5. Gelenk nach Anspruch 2, **dadurch gekennzeichnet,** daß die beiden Funktionsflächen konkav ausgebildet sind und ihre Rotationszentren $M_1$ und $M_2$ im Druckverteilungskörper liegen und ihre Gelenkachsenbahn den Radius $R = R_2 + R_1 - D$ besitzt.

6. Gelenk nach Anspruch 2, **dadurch gekennzeichnet,** daß eine Funktionsfläche eines Gelenkteils konvex und die andere Funktionsfläche (23) des Gelenkteils konkav ausgebildet ist und das Rotationszentrum $M_2$ des Gelenkteils mit der konvexen Funktionsfläche in diesem und das Rotationszentrum $M_1$ des Gelenkteils mit der konkaven Funktionsfläche im Druckverteilungskörper liegt und ihre Gelenkachsenbahn einen Radius $R = R_2 - R_1 + D$ besitzt.

7. Gelenk nach Anspruch 4, **dadurch gekennzeichnet,** daß die minimale Dicke D des Druckverteilungskörpers (25) kleiner/gleich 50% des kleineren Radius $R_1$ beträgt.

**Claims**

1. Artificial joint for the replacement, in particular, of human joints, comprising at least two joint parts (21, 22) with spherical functional surfaces which move relative to each other, the relationships of the curvature relative to each other of the functional surfaces (23, 24) which have a circular sectional contour being convex-convex, convex-concave or concave-concave and the joint geometry being determined by a linkage with two joint axes running through the centres of rotation $M_1$ and $M_2$ of the functional surfaces with the radii $R_1$ and $R_2$, where $R_1$ is the radius of the circular sectional contour of the functional surface with the centre point $M_1$ and $R_2$ is the radius of the circular sectional contour of the functional surface with the centre point $M_2$, and a compression distribution element (25) being arranged between the two functional surfaces (23, 24) of the joint parts (21, 22), the sliding surfaces (26, 27), bearing against the functional surfaces, of which compression distribution element have a curvature adapted to the functional surfaces, characterised in that the sliding surfaces (26, 27) of the compression distribution element (25) are of the same size, or in that there is a difference between the surface sizes of the two sliding surfaces (26, 27) of the compression distribution element (25) of at most 5 - 10 %.

2. Joint according to Claim 1, characterised in that the sliding surfaces (26, 27) are highly polished.

3. Joint according to Claim 2, characterised in that the functional surfaces (23, 24) of the joint parts (21, 22) are convexly shaped and their centres of rotation $M_1$ and $M_2$ lie in the associated joint parts (21, 22) and the path of their joint axes has a radius $R = R_1 + R_2 + D$.

4. Joint according to Claim 2, characterised in that respectively one functional surface (24) of one joint part (22) is convexly shaped and the other functional surface (23) of the joint part (21) is concavely shaped and their centres of rotation $M_1$ and $M_2$ lie inside the joint part with the convex functional surface and the path of their joint axes has a radius $R = R_2 - R_1 - D$, where $R_2$ is greater than the sum of $R_1$ and D, thereby ensuring a stable, overlapping, dimerous linkage.

5. Joint according to Claim 2, characterised in that the two functional surfaces are concavely shaped and their centres of rotation $M_1$ and $M_2$ lie in the compression distribution element and the path of their joint axes has the radium $R = R_2 + R_1 - D$.

6. Joint according to Claim 2, characterised in that one functional surface of one joint part is convexly shaped and the other functional surface (23) of the joint part is concavely shaped and the centre of rotation $M_2$ of the joint part with the convex functional surface lies in this joint part and the centre of rotation $M_1$ of the joint part with the concave functional surface lies in the compression distribution element and the path of their joint axes has a radius $R = R_2 - R_1 + D$.

7. Joint according to Claim 4, characterised in that the minimum thickness D of the compression distribution element (25) is less than or equal to 50% of the smaller radius $R_1$.

**Revendications**

1. Articulation artificielle utilisée en particulier dans les articulations humaines, comprenant au moins deux éléments (21, 22) munis de surfaces fonctionnelles sphériques mobiles entre elles, la courbure entre elles des surfaces fonctionnelles (23, 24) qui présentent un contour de coupe circulaire étant soit convexe-convexe, soit convexe-concave, soit concave-concave, et la géométrie d'articulation étant déterminée par une chaîne d'articulation comportant deux axes d'articulation s'étendant à travers les centres de rotation $M_1$ et $M_2$ des surfaces fonctionnelles de rayons $R_1$ et $R_2$, $R_1$ étant le rayon du contour de coupe circulaire de la surface fonctionnelle dont le centre est $M_1$, et $R_2$, étant le rayon du contour de coupe circulaire de la surface fonctionnelle dont le centre est $M_2$, un corps de répartition de la pression (25) étant disposé entre les deux surfaces fonctionnelles (23, 24) des éléments d'articulation (21, 22) dont les surfaces de glissement (26, 27), appliquées aux surfaces fonctionnelles, présentent une courbure adaptée aux surfaces fonctionnelles, caractérisée en ce que les surfaces de glissement (26, 27) du corps de répartition de la pression (25) sont de dimensions identiques, ou qu'il existe une différence de 5-10% maximum entre les dimensions de surface des deux surfaces de glissement (26, 27) du corps de répartition de la pression (25).

2. Articulation selon la revendication 1, caractérisée en ce que les surfaces de glissement (26, 27) présentent un poli miroir.

3. Articulation selon la revendication 2, caractérisée en ce que les surfaces fonctionnelles (23, 24) des éléments d'articulation (21, 22) ont une forme convexe, que leurs centres de rotation $M_1$ et $M_2$ sont situés dans l'élément d'articulation associé (21, 22), et que le trajet de leur axe d'articulation présente un rayon $R = R_1 + R_2 + D$.

4. Articulation selon la revendication 2, caractérisée en ce qu'une surface fonctionnelle respective (24) d'un élément d'articulation (22) est convexe et que l'autre surface fonctionnelle (23) de l'élément d'articulation (21) est concave, que leurs centres de rotation $M_1$ et $M_2$ sont situés à l'intérieur de l'élément d'articulation présentant la surface fontionnelle convexe, et que leur trajet d'axe d'articulation présente un rayon de $R = R_2 - R_1 - D$, $R_2$ étant supérieur à la somme de $R_1$ et de D, ce qui assure une chaîne dimère stable recouverte.

5. Articulation selon la revendication 2, caractérisée en ce que les deux surfaces fonctionnelles ont une forme concave, que leurs centres de rotation $M_1$ et $M_2$ sont situés dans le corps de répartition de la pression, et que leur trajet d'axe d'articulation présente le rayon $R = R_2 + R_1 - D$.

6. Articulation selon la revendication 2, caractérisée en ce que l'une des surfaces fonctionnelles de l'un des éléments d'articulation est convexe, que l'autre surface fonctionnelle (23) de l'élément d'articulation est concave, que le centre de rotation $M_2$ de l'élément d'articulation qui présente la surface fonctionnelle convexe est situé dans cet élément, que le centre de rotation $M_1$ de l'élément d'articulation qui présente la surface fonctionnelle concave est situé dans le corps de répartition de la pression, et que son trajet d'axe d'articulation présente un rayon $R = R_2 - R_1 + D$.

7. Articulation selon la revendication 4, caractérisée en ce que l'épaisseur minimum D du corps de répartition de la pression (25) est inférieure ou égale à 50% du petit rayon $R_1$.